# EUROPÄISCHE PATENTSCHRIFT

(11) **EP 4 615 343 B1**
(45) Veröffentlichungstag und Bekanntmachung des Hinweises auf die Patenterteilung: **20.05.2026**
(21) Anmeldenummer: 23804780.7
(22) Anmeldetag: 06.11.2023
(51) Int. Cl.: A61B 17/16, A61B 17/34

(54) **VORRICHTUNG ZUM DURCHDRINGEN EINER ANATOMISCHEN STRUKTUR**
APPARATUS FOR PENETRATING THROUGH AN ANATOMICAL STRUCTURE
APPAREIL DE PÉNÉTRATION À TRAVERS UNE STRUCTURE ANATOMIQUE

(30) Priorität: 07.11.2022 AT 2102022
(43) Veröffentlichungstag der Anmeldung: 17.09.2025
(73) Patentinhaber: Medizinische Universität Wien, 1090 Wien (AT)
(72) Erfinder: SCHWINDT, Eva, 3021 Pressbaum (AT); UNGER, Ewald, 1210 Wien (AT)
(74) Vertreter: SONN Patentanwälte GmbH & Co KG
(86) Internationale Anmeldenummer: PCT/IB2023/061156
(87) Internationale Veröffentlichungsnummer: WO 2024/100513

(56) Entgegenhaltungen:
- WO-A1-2011/123703
- DE-A1- 102008 032 704
- KR-B1- 101 064 079
- US-A1- 2005 131 345
- US-A1- 2021 322 055

## Beschreibung

Vorrichtung zum Durchdringen einer anatomischen Struktur, die zum Erkennen des Durchdringens einer anatomischen Struktur und zum automatischen Stoppen eines Durchdringungsvorgangs bei Erkennen des Durchdringens der anatomischen Struktur ausgelegt ist, wobei die Vorrichtung entlang einer Längsachse der Vorrichtung ein proximales Handgerät und einen daran abnehmbar festgelegten distalen Aufsatz umfasst.

Eine derartige Vorrichtung ist beispielsweise aus EP 2 671 531 B1 bekannt geworden. In diesem Dokument ist ein medizinisches Bohrwerkzeug offenbart, bei dem ein Bohrer entlang seiner Längsachse von einem verschieblichen Fühler durchsetzt ist. Wenn mit diesem Werkzeug eine Knochenwand mit dahinter liegendem, weicherem Gewebe durchbohrt wird, dringt der Fühler in das weichere Gewebe hinter der Knochenwand ein und löst dadurch ein Signal zum Stoppen des Bohrwerkzeugs aus. Auf diese Weise werden Verletzungen des hinter der Knochenwand liegenden weicheren Gewebes vermieden. Insbesondere ist dieses Bohrwerkzeug für den Einsatz im dentalen Bereich konzipiert.

Das in EP 2 671 531 B1 offenbarte Bohrwerkzeug hat eine Reihe von Nachteilen. So ist der Bohrer mit dem darin angeordneten verschieblich gelagerten Fühler relativ aufwendig und kann nur schlecht zur Wiederverwendung sterilisiert werden. Aus diesem Grund ist der Einsatz des Bohrwerkzeugs der EP 2 671 531 B1 relativ teuer und kann daher aus ökonomischen Gründen nicht massenhaft erfolgen.

Dokument WO 2011/123703 A1 offenbart Vorrichtungen und Verfahren zum Erzeugen einer Bohrung in Knochen. Die beschriebenen Vorrichtungen und Verfahren umfassen das Steuern des Antriebs und das Messen der Wirkung eines Werkzeugs.

Weitere chirurgische Vorrichtungen sind aus US 2021/322055 A1, DE 10 2008 032704 A1, KR 101 064 079 B1 und US 2005/131345 A1 bekannt.

In der Erwachsenenmedizin und bei Kindern jenseits des Neugeborenenalters ist der intraossäre Zugang in Notfallsituationen ein gut etabliertes, sicheres und gut untersuchtes Werkzeug zur Gabe von Notfallmedikamenten und wird als Zugang der ersten Wahl in internationalen Reanimations-Leitlinien empfohlen. Für die Reanimation von Neugeborenen gibt es hierzu bisher jedoch nur lückenhafte Erfahrungen und Daten. Der Anwendung des intraossären Zugangs in dieser Altersgruppe stehen großteils technische Schwierigkeiten mit den bisher erhältlichen Knochennadeln entgegen, die nicht für die Dimensionen bei Neugeborenen entwickelt wurden. Die größte Schwierigkeit besteht darin, während des Durchdringungsvorgangs beziehungsweise Bohrvorgangs durch die vordere Knochenwand ein Verletzen der Rückseite des Knochens zu vermeiden. Der Anwender muss demnach mit größter Vorsicht den Durchdringungsvorgangs oder Bohrvorgang sofort manuell stoppen, sobald der Markraum des Knochens erreicht ist. Durch die geringe Größe des Neugeborenen-Knochens (für den intraossären Zugang wird der Unterschenkelknochen empfohlen) von nur 7-8 mm kommt es bei nur gering zu starker Kraftanwendung zu einer ungewollten Durchbohrung auch der Rückseite und damit in der Folge zu einem Austritt der applizierten Medikamente in das umliegende Gewebe. Dieses sogenannte Paravasat kann im Langzeitverlauf zu Gewebedestruktion, Kompartmentsyndrom und im schlimmsten Fall zur Notwendigkeit der Amputation des Beines führen. Noch entscheidender ist jedoch, dass in der lebensbedrohlichen Situation lebensrettende Medikamente nicht im Kreislauf des Kindes ankommen können.

Es ist daher Aufgabe der vorliegenden Erfindung, eine Vorrichtung der eingangs genannten Art dahingehend weiterzubilden, dass das Durchdringen der anatomischen Struktur und insbesondere des Knochens auch mit besonders dünnen Bohrern beziehungsweise anderen Durchdringungsspitzen wie Kanülen zuverlässig erkannt werden und ein automatischer Stopp des Vorgangs erfolgen kann. Des Weiteren soll die erfindungsgemäße Vorrichtung kostengünstig in der Anwendung sein und insbesondere mit besonders kostengünstigen Spitzen beziehungsweise Bohrern oder anderen Durchdringungsspitzen wie Kanülen zu verwenden sein, um nach dem Durchdringen der anatomischen Struktur das Einbringen von Medikamenten und Flüssigkeiten in den Markraum zur Therapie ermöglichen.

Zur Lösung dieser Aufgabe ist die Vorrichtung der eingangs genannten Art erfindungsgemäß dadurch gekennzeichnet, dass der Aufsatz eine entlang der Längsachse verschiebbar gelagerte Durchdringungsspitze aufweist, die über eine Schubstange mit einem Linearantrieb im Handgerät in Wirkverbindung steht, wobei die Schubstange durch eine federnde Lagerung vorzugsweise an ihrem proximalen Ende zum Einfedern in die proximale Richtung ausgelegt ist und die Vorrichtung Mittel zum Erkennen eines Ausfederns der Schubstange in die distale Richtung bei Erkennen des Durchdringens der anatomischen Struktur aufweist.

Es wird somit eine Durchdringungsvorrichtung geschaffen, bei der jede Art von Durchdringungsspitze eingesetzt werden kann und dennoch eine zuverlässige Erkennung des Durchdringens einer anatomischen Struktur wie einer Knochenwand erfolgen kann, um den Vorgang beim Durchdringen automatisiert zu stoppen. Die gemäß der vorliegenden Erfindung in herkömmlicher Weise ausgebildete Durchdringungsspitze ist kostengünstig, da in der Durchdringungsspitze selbst keinerlei Vorkehrungen zum Erkennen des Durchdringens der anatomischen Struktur getroffen sind und die Durchdringungsspitze daher als einfacher Bohrer oder auch nur als Kanüle ausgebildet sein kann. Die Durchdringungsspitze kann daher ohne Weiteres als Einmal-Artikel ausgebildet sein, um höchste Anforderungen an die Hygiene und Anwenderfreundlichkeit zu erfüllen. Die Durchdringungsspitze weist anders als im Stand der Technik einen einfachen Aufbau auf und ist daher nicht den entsprechenden Beschränkungen hinsichtlich einer Verringerung des Durchmessers der Durchdringungsspitze zum Einsatz bei der intraossären Medikamentengabe in der Neonatologie unterworfen. Insbesondere kann die Durchdringungsspitze daher beispielsweise mit Durchmessern von 20 Gauge (0,9 mm Außendurchmesser nach EN ISO 9626) und darunter hergestellt werden, wie dies einer bevorzugten Ausführungsform der vorliegenden Erfindung entspricht.

Bevorzugt ist die federnde Lagerung von einem Magnetelement im proximalen Endbereich der Schubstange und einem antiseriell dazu angeordneten Magnetelement im distalen Endbereich des Linearantriebs gebildet. Durch die antiseriell angeordneten Magnetelemente, das heißt durch die mit gleichsinnigen Polen zueinander gerichteten Magnetelemente, wird eine federnde Lagerung bereitgestellt, die einen einfachen Aufbau der erfindungsgemäßen Vorrichtung erlaubt. Gleichzeitig wird eine auch über längere Zeit konstant definierte Federkraft für das Erkennen des Durchdringens beispielsweise der Knochenwand der Tibia eines Neugeborenen durch Erkennen des Ausfederns der Schubstange bereitgestellt, die in mechanischer Hinsicht überaus robust und im Vergleich zum Einsatz herkömmlicher Federn wenig fehleranfällig ist.

Bevorzugt ist die Durchdringungsspitze als Bohrer und die Schubstange als Antriebswelle ausgebildet, wobei die Antriebswelle mit einem Bohrantrieb in Wirkverbindung steht und der Bohrantrieb in dem Handgerät entlang der Längsachse verschiebbar gelagert und durch den Linearantrieb zur Verschiebung angetrieben ist. Auf diese Weise kann das Durchdringen der Knochenwand nicht nur durch Vorschub einer Spitze durch die Knochenwand mit Hilfe des Linearantriebs erfolgen, sondern das Durchdringen der Knochenwand wird zusätzlich durch die spanende Wirkung des durch den Bohrantrieb zur Rotation angetriebenen Bohrers als Durchdringungsspitze unterstützt. Dies bedeutet, dass der Bohrantrieb zum Durchdringen des Knochens und zur Eröffnung des Markraums in dem Handgerät entlang der Längsachse verschiebbar angetrieben ist, sodass bei auf die Haut des Patienten aufgesetztem distalen Aufsatz der Vorschub der als Bohrer ausgeführten Durchdringungsspitze automatisiert vonstatten geht und daher eventuelle Ungeschicklichkeiten der behandelnden Person eliminiert werden.

Bevorzugt ist hierbei die federnde Lagerung der Antriebswelle von einem Magnetelement im proximalen Endbereich der Antriebswelle und einem antiseriell dazu angeordneten Magnetelement im distalen Endbereich des Bohrantriebs gebildet. Hierbei werden dieselben Vorteile erzielt wie vorstehend beschrieben bei der federnden Lagerung der Schubstange mit Hilfe von Magnetelementen.

Die federnde Lagerung der Schubstange beziehungsweise der Antriebswelle kann auch auf andere Weise als durch Magnetelemente realisiert sein, wie z.B. durch eine mechanische Feder. Wesentlich für die grundsätzlich beliebige Art der federnden Lagerung ist es hierbei im Rahmen der Erfindung, dass die Schubstange beziehungsweise die Antriebswelle gegen einen gewissen Widerstand nach proximal einfedern und nach distal ausfedern kann, wobei es grundsätzlich unerheblich ist, ob die Federbewegung gedämpft oder ungedämpft ist oder ob die Federkraft entlang des Federwegs in irgendeiner Weise zunimmt oder abnimmt.

Gemäß einer bevorzugten Ausführungsform der vorliegenden Erfindung sind die Mittel zum Erkennen des Ausfederns der Schubstange von einem Sensor zum Messen des Magnetfelds der Magnetelemente, insbesondere von einem im Bereich des von den Magnetelementen gebildeten Magnetfelds angeordneten Hall-Sensor, gebildet. Auf diese Weise wird die Art der Realisierung der federnden Lagerung gleichzeitig für das Erkennen des Ausfederns der Schubstange beziehungsweise der als Antriebswelle ausgebildeten Schubstange genutzt, sodass mit geringstmöglichem Aufwand mit einem Sensor das Ausfedern der Schubstange beziehungsweise der Antriebswelle und damit das Durchdringen der anatomischen Struktur erkannt und der Durchdringungsvorgang oder bevorzugt der Bohrvorgang z.B. durch Stoppen des Linearantriebs und/oder Bohrantriebs ohne das Zutun der behandelnden Person automatisch beendet werden kann, um das ungewollte Durchdringen weiterer Strukturen zu verhindern.

Zur Verhinderung des vollständigen Durchdringens beispielsweise der Tibia eines Neugeborenen kann es hierbei vorgesehen sein, dass der Linearantrieb zum automatischen Stoppen des Durchdringungsvorgangs bei Erkennen des Durchdringens der anatomischen Struktur durch Stoppen des Linearantriebs ausgelegt ist, wie dies einer bevorzugten Ausführungsform der vorliegenden Erfindung entspricht. Somit gestattet es die vorliegende Erfindung, den Durchdringungsvorgang vollständig automatisiert durchzuführen, indem der Aufsatz auf die Haut des Patienten aufgesetzt wird, die erfindungsgemäße Vorrichtung in Betrieb gesetzt und der Knochen zur Verabreichung einer Medikamentendosis durch Vorschub der Durchdringungsspitze bis in den Markraum eröffnet wird. Sobald die erfindungsgemäße Vorrichtung das Durchdringen des Knochens erkennt, wird der Linearantrieb gestoppt oder es werden im Falle des zusätzlichen Vorsehens eines Bohrantriebs vorzugsweise sowohl der Bohrantrieb als auch der Linearantrieb gestoppt und der Bohrer beziehungsweise die Durchdringungsspitze ragt in den Markraum des Knochens. Eine Durchdringung auch der hinteren Knochenwand wird auf diese Weise sicher verhindert, sodass auch in unter Umständen emotional schwierigen Situationen bei lebensrettenden Maßnahmen an Neugeborenen und auch Frühgeborenen kein Risiko besteht, die vorgesehenen Medikamente außerhalb des Knochens abzugeben.

Ergänzend kann es gemäß einer bevorzugten Ausführungsform vorgesehen sein, dass ein Sensor ein erneutes Einfedern der Schubstange beziehungsweise der Antriebswelle erfasst. Dies kann als Kontakt der Durchdringungsspitze zur hinteren Knochenwand interpretiert werden, sodass ein Notstopp der erfindungsgemäßen Vorrichtung ausgeführt werden kann.

Gemäß einer bevorzugten Ausführungsform der vorliegenden Erfindung ist es vorgesehen, dass die Schubstange zwischen ihrem proximalen Ende und dem Aufsatz quer zur Längsachse in einen distalen Teil im Aufsatz und einen proximalen Teil im Handgerät geteilt ist. Auf diese Weise kann der proximale Teil der Schubstange mit der Magnetscheibe beim Abnehmen des Aufsatzes im Handgerät verbleiben, während nur der distale Teil der Schubstange beim Wechseln des Aufsatzes abgenommen und in aller Regel mit dem Aufsatz verworfen wird. Die ausreichend drehfeste und dennoch lösbare Verbindung zwischen dem proximalen Teil der Schubstange und dem distalen Teil der Schubstange kann auf jede denkbare Art erfolgen.

Beispielsweise weisen der proximale und der distale Teil der Schubstange an ihren zueinander weisenden Enden einander entsprechende Eingriffsmittel auf.

Gemäß einer bevorzugten Ausführungsform der vorliegenden Erfindung ist die Durchdringungsspitze hinter einer distalen Spitze mit einem Hohlraum ausgebildet und weist im Bereich des Hohlraums zumindest eine Durchbrechung zur Abgabe eines Arzneimittels aus dem Hohlraum auf, wobei die Durchdringungsspitze bevorzugt mittels eines Luer/Lock-Anschlusses mit dem distalen Teil der Schubstange in Wirkverbindung steht. Gemäß dieser bevorzugten Ausführungsform kann die Durchdringungsspitze durch Zusammendrücken und Anschleifen einer herkömmlichen Injektionskanüle und durch Anbohren des Lumens der Kanüle hergestellt werden. Dies ermöglicht die Abgabe des Arzneimittels direkt über die in den Knochen eingebrachte Durchdringungsspitze und es muss nach dem Setzen der Bohrung keine eigene Kanüle in den Knochen eingebracht werden. Wenn die Durchdringungsspitze über einen Luer/Lock-Anschluss an die Antriebswelle angeschlossen ist, kann jedes mit diesem gängigen Anschlusssystem ausgestattete Infusionssystem nach dem Abnehmen des Handgeräts an die Durchdringungsspitze angeschlossen werden. Auf diese Weise ist die erfindungsgemäße Vorrichtung unmittelbar mit der Infusionsausstattung einer Klinik kompatibel und kann höchst flexibel eingesetzt werden.

Gemäß einer bevorzugten Ausführungsform der vorliegenden Erfindung kann der Aufsatz aus einem transparenten Kunststoff gebildet sein und/oder zumindest eine seitliche Durchbrechung aufweisen. Dies dient dazu, die Durchdringungsspitze und die Bohrstelle oder Einstichstelle am Patienten für die behandelnde Person einsehbar zu halten.

Bevorzugt ist der Bohrer zu einer zyklischen Änderung der Drehrichtung antreibbar, wodurch verhindert werden kann, dass sich die Haut, Weichteile oder chirurgische Abdeckungen um den Bohrer wickeln können, woraus unangenehme und unter Umständen auch kritische Situationen eintreten könnten. Hierbei wird sichergestellt, dass der Bohrer keine vollständige Umdrehung vollführt, sondern dass die Drehrichtung vor Erreichen einer vollständigen Umdrehung umgekehrt wird.

Für eine einfache und schnelle Bedienbarkeit ist die vorliegende Erfindung bevorzugt dahingehend weitergebildet, dass der Aufsatz an dem Handgerät durch eine Rastverbindung, eine Drehverbindung oder einen Bajonettverschluss festlegbar ist.

Während es bei der vorliegenden Erfindung vorgesehen ist, den Aufsatz mit der darin befindlichen Durchdringungsspitze und gegebenenfalls dem distalen Teil der Schubstange als Einmalartikel auszubilden, um die nötige Sterilität der Durchdringungsspitze zu gewährleisten, kann es immer wieder notwendig sein, das Handgerät zu reinigen oder auch zu sterilisieren. Aus diesem Grund kann das Handgerät hitzefest und druckfest ausgebildet sein, wie dies einer bevorzugten Ausführungsform der vorliegenden Erfindung entspricht.

Insbesondere um eine rasche und sichere Bedienbarkeit der erfindungsgemäßen Vorrichtung zu gewährleisten und um eine einmal zur Infusion gesetzte Durchdringungsspitze, die mit einer verbreiterten Spitze hinter der Knochenwand bis zu einem gewissen Grad festgelegt ist, beim Abnehmen des Handgeräts nicht unnötig zu bewegen und das Durchdringungsloch im Knochen dadurch unnötig zu vergrößern und den Sitz der Durchdringungsspitze dadurch zu beeinträchtigen, kann die vorliegende Erfindung bevorzugt dahingehend weitergebildet sein, dass die Vorrichtung eine Abwurfvorrichtung zum Trennen der Durchdringungsspitze vom Aufsatz aufweist. Eine derartige Abwurfvorrichtung kann auf jede erdenkliche Art ausgeführt sein und führt beim Betätigen zu einem Abdrücken der Durchdringungsspitze von der Schubstange. Das Handgerät wird auf diese Weise schonend freigegeben und die Durchdringungsspitze verbleibt zur Infusion in den Markraum im Knochen. In der Folge muss nur noch ein Infusionssystem beispielsweise über den Luer/Lock-Anschluss an die Durchdringungsspitze angeschlossen werden.

Die Erfindung wird nachfolgend anhand eines in der Zeichnung dargestellten Ausführungsbeispiels näher erläutert. In dieser zeigen Figur 1 eine Gesamtansicht der erfindungsgemäßen Vorrichtung im Schnitt, die Figuren 2a bis 2c den Aufsatz in verschiedenen Ansichten, Figur 3 ein Detail der federnden Lagerung einer als Antriebswelle ausgebildeten Schubstange in einem eingefederten Zustand während des Bohrens, Figur 4 ein Detail der federnden Lagerung der Antriebswelle in einem ausgefederten Zustand beim Stoppen des Bohrvorgangs, Figur 5 eine Detailansicht des vorderen Teils der erfindungsgemäßen Vorrichtung im Schnitt in einem eingefederten Zustand während des Bohrens, Figur 6 eine Detailansicht des vorderen Teils der erfindungsgemäßen Vorrichtung im Schnitt in einem ausgefederten Zustand beim Stoppen des Bohrvorgangs und die Figuren 7 a und 7b Detailansichten einer als Bohrer ausgebildeten Durchdringungsspitze der erfindungsgemäßen Vorrichtung.

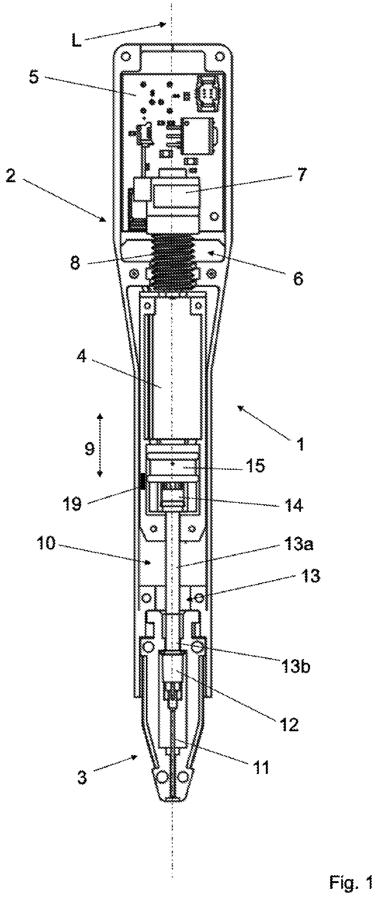

In Figur 1 ist die erfindungsgemäße Vorrichtung mit dem Bezugszeichen 1 bezeichnet. Die Vorrichtung 1 besteht im Wesentlichen aus einem proximalen Handgerät 2 und einem daran abnehmbar festgelegten distalen Aufsatz 3. Die Längsachse der erfindungsgemäßen Vorrichtung ist mit L bezeichnet. Im Handgerät 2 sind ein Bohrantrieb 4 und eine Steuereinheit 5 für den Bohrantrieb 4 und ein Linearantrieb 6 aufgenommen. Der Bohrantrieb 4 kann durch den Linearantrieb 6, der im Wesentlichen aus einem Motor 7 mit Spindel 8 besteht, entlang der Längsachse L in Richtung des Doppelpfeils 9 in einer Führung 10 verschoben werden, um die als Bohrer 11 ausgebildete Durchdringungsspitze 11 in der axialen Richtung nach distal voran zu treiben. Der Bohrer 11 ist mittels eines Luer/Lock-Anschlusses 12 an der als Antriebswelle ausgeführten Schubstange 13 befestigt, wobei die Antriebswelle 13 aus einem proximalen Teil 13a im Handgerät 2 und einem distalen Teil 13b im Aufsatz 3 besteht.

Die Antriebswelle 13, insbesondere deren proximaler Teil 13a ist durch die Wirkung zweier Magnetelemente 14 und 15 federnd gelagert, wobei die federnde Lagerung in Figur 1 in ausgefedertem Zustand dargestellt ist. Im Bereich der Magnetelemente 14 und 15 ist ein Hall-Sensor 19 angeordnet, der mit der Steuereinheit 5 verbunden ist, um Veränderungen des Magnetfelds der beiden Magnetelemente 14 und 15 zu registrieren. Das Signal dieses Sensors 19 wird in der Steuereinheit 5 zum Stoppen des Bohrvorgangs ausgewertet.

In den weiteren Figuren sind gleiche oder einander entsprechende Teile mit gleichen Bezugszeichen bezeichnet.

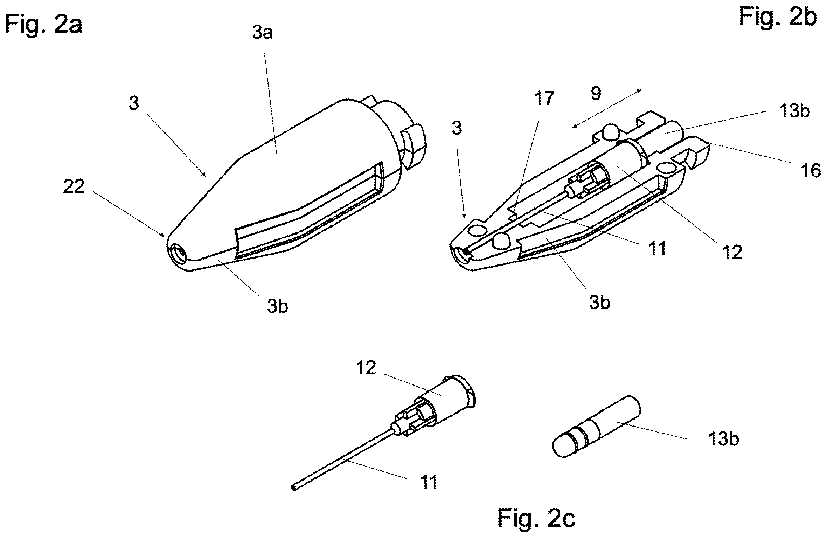

In Figur 2a ist der Aufsatz 3 als aus zwei Schalen 3a und 3b zusammengesetzter Bauteil dargestellt, wobei die Schalen 3a und 3b auch aus transparentem Material hergestellt sein können, um den Blick auf den Bohrer 11 freizugeben. Mit dem Bezugszeichen 22 ist das distale Ende des Aufsatzes 3 bezeichnet. Der Bohrer 11 (Figur 2b) ist im Aufsatz 3 entlang des Doppelpfeils 9 (wie in Figur 1) zur Verschiebung im Aufsatz 3 gelagert und kommt gegen einen proximalen Anschlag 16 sowie einen distalen Anschlag 17 zur Anlage. Der Bohrer 11 ist mit dem Luer/Lock-Anschluss 12 am distalen Ende des distalen Teils 13b der Antriebswelle 13 aufgesteckt (Figur 2c), wodurch ein ausreichend hohes Drehmoment übertragbar ist, um beispielsweise den Tibiaknochen eines Neugeborenen im Bereich des Tibiakopfes zu bohren.

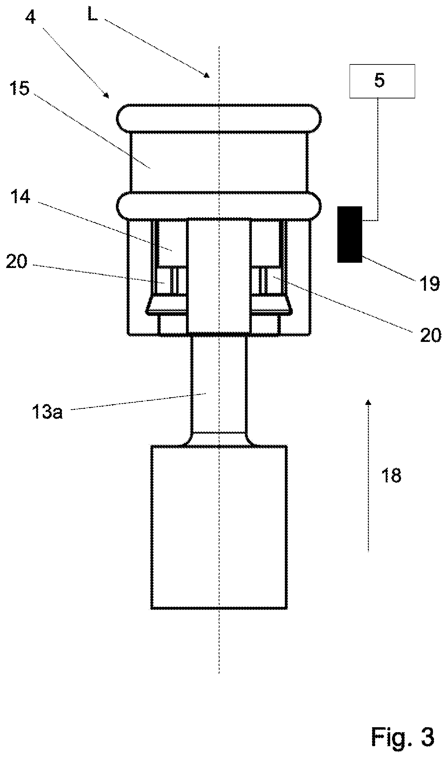

In Figur 3 ist ein Teil der Antriebswelle 13 beziehungsweise ein Teil des proximalen Teils 13a vergrößert dargestellt. Die Antriebswelle 13a weist am proximalen Ende ein Magnetelement 14 auf, welches sich aufgrund einer antiseriellen Anordnung gegenüber einem Magnetelement 15 des in Figur 3 nur ebenfalls nur teilweise dargestellten Bohrantriebs 4 vom Bohrantrieb 4 federnd abstößt. Wenn nun ausreichend harte Strukturen, wie beispielsweise die Kortikalis der Tibia eines Neugeborenen gebohrt werden, bleibt die Antriebswelle 13 beziehungsweise 13a in Richtung des Pfeils 18 eingefedert, was vom schematisch dargestellten und mit dem Bezugszeichen 19 bezeichneten Sensor zusammen mit der Steuereinheit 5 als eingefederter Zustand interpretiert wird. Mit 20 sind Führungsplatten bezeichnet, die in eine entsprechende Nut der Antriebswelle 13a eingreifen, um ein Drehmoment vom Bohrantrieb 4 auf die Antriebswelle 13 beziehungsweise 13a zu übertragen.

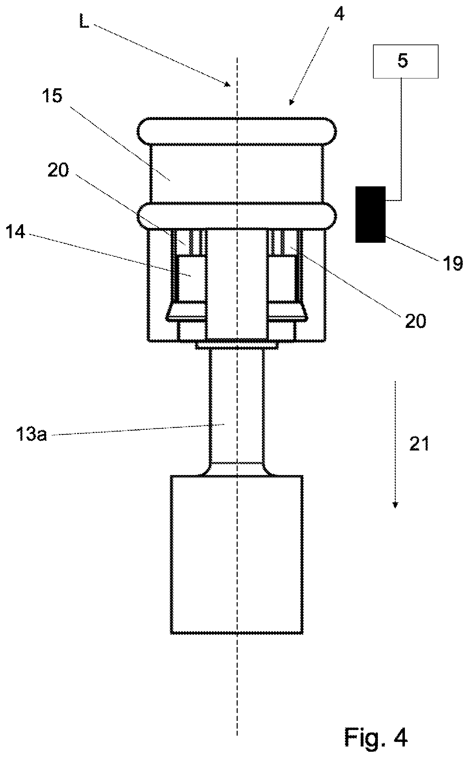

In der Darstellung nach Figur 4 ist nun die Antriebswelle 13 beziehungsweise deren proximaler Teil 13a in einem ausgefederten Zustand dargestellt, der dann eintritt, wenn der Bohrer 11 nach dem Durchdringen der Kortikalis beziehungsweise der Knochenwand im Markraum des Knochens einen geringeren Widerstand in der Längsrichtung L erfährt. Die Antriebswelle 13 beziehungsweise 13a federt dann in Richtung des Pfeils 21 aus, was vom Sensor 19 zusammen mit der Steuereinheit 5 wiederum als ausgefederter Zustand interpretiert wird. Im ausgefederten Zustand werden der Bohrantrieb 4 und der Linearantrieb 6 gestoppt, sodass kein weiteres Eindringen des Bohrers 11 in den Knochen erfolgt.

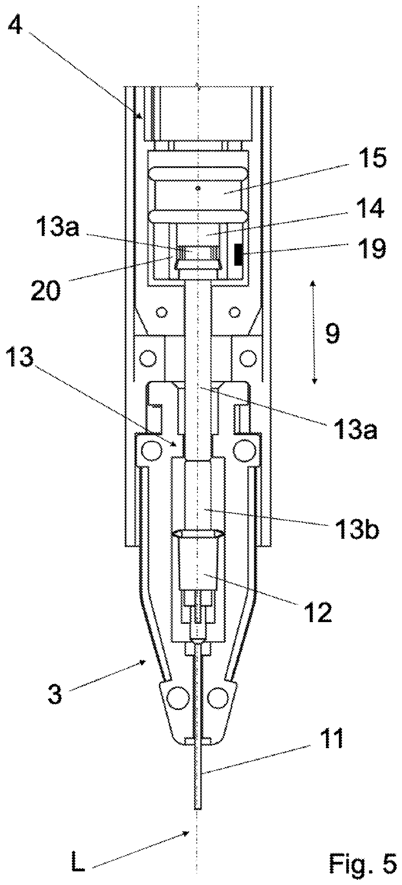

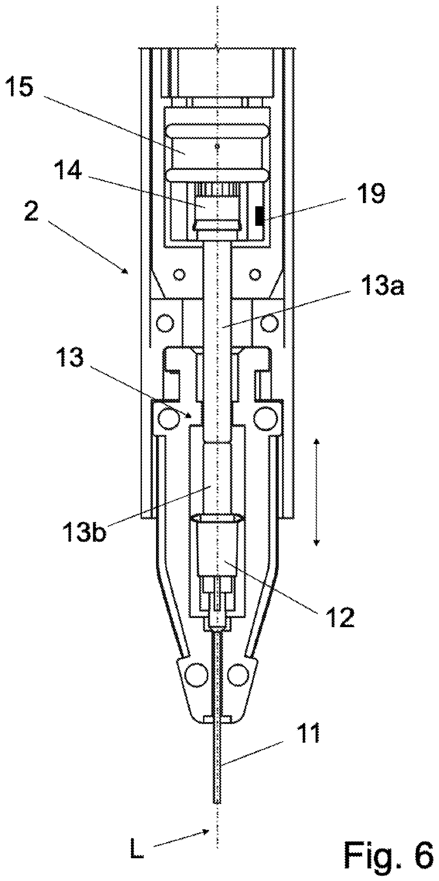

Diese Funktionsweise ist in den Figuren 5 und 6 noch einmal verdeutlicht. In Figur 5 ist die Antriebswelle 13 im eingefederten Zustand dargestellt, sodass die Magnetelemente 14 und 15 aneinander anliegen. Dies kann vom Sensor 19 zusammen mit der Steuereinheit 5 als Zustand interpretiert werden, bei dem der Bohrvorgang aufrechterhalten wird. Der Bohrantrieb 4 versetzt die Antriebswelle 13 und damit den Bohrer 11 in Rotation und der in Figur 5 nicht dargestellte Linearantrieb verschiebt den Bohrantrieb 4 in die distale Richtung, sodass der Bohrer 11 in den Knochen eindringt. Wenn der Bohrer 11 durch die Knochenwand hindurch in den weichen Markraum getreten ist, federt die Antriebswelle aus (Figur 6), sodass das Magnetelement 14 nun weiter vom Magnetelement 15 entfernt ist. Dies bringt eine deutliche Änderung des Magnetfelds mit sich, was vom Sensor 19 zusammen mit der Steuereinheit 5 als Durchdringen der anatomischen Struktur interpretiert wird. Der Bohrantrieb 4 und der Linearantrieb 6 werden unmittelbar gestoppt und der Bohrer 11 kann vom Aufsatz 3 beziehungsweise von der Antriebswelle 13a abgedrückt werden.

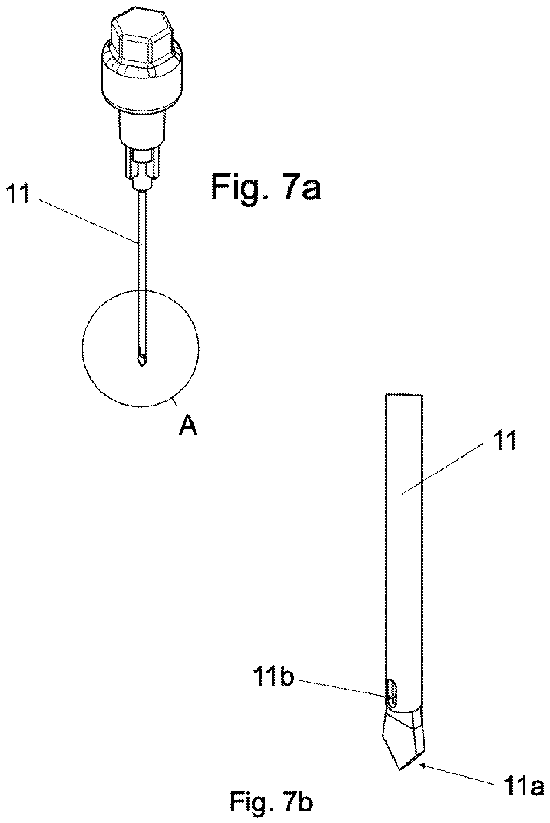

In Figur 7a ist zu erkennen, dass der Bohrer 11 im Grunde einer Injektionskanüle entspricht, die durch Abflachen der Spitze und Fenestrieren der zylindrischen Wandung zu einem Bohrer umgearbeitet wurde, über den nach dem Bohrvorgang die Medikamentengabe in den Markraum eines Knochens erfolgen kann. Die Spitze des Bohrers 11 ist in Figur 7b mit dem Bezugszeichen 11a bezeichnet. Hinter der Spitze 11a, das heißt proximal davon, ist eine Durchbrechung 11b zu erkennen, die in den Hohlraum im Inneren des Bohrers 11 reicht. Wenn der Bohrer tief genug im Markraum liegt, kann über diese Durchbrechung 11b eine Medikamentendosis abgegeben werden.

## Patentansprüche

1. Vorrichtung (1) zum Durchdringen einer anatomischen Struktur, die zum Erkennen des Durchdringens einer anatomischen Struktur und zum automatischen Stoppen eines Durchdringungsvorgangs bei Erkennen des Durchdringens der anatomischen Struktur ausgelegt ist, wobei die Vorrichtung (1) entlang einer Längsachse (L) der Vorrichtung (1) ein proximales Handgerät (2) und einen daran abnehmbar festgelegten distalen Aufsatz (3) umfasst, wobei der Aufsatz (3) eine entlang der Längsachse (L) verschiebbar gelagerte Durchdringungsspitze (11) aufweist, die über eine Schubstange (13) mit einem Linearantrieb (6) im Handgerät (2) in Wirkverbindung steht, **dadurch gekennzeichnet, dass** die Schubstange (13) durch eine federnde Lagerung vorzugsweise an ihrem proximalen Ende zum Einfedern in die proximale Richtung ausgelegt ist und die Vorrichtung (1) Mittel zum Erkennen eines Ausfederns der Schubstange in die distale Richtung bei Erkennen des Durchdringens der anatomischen Struktur aufweist.

2. Vorrichtung nach Anspruch 1, **dadurch gekennzeichnet, dass** die federnde Lagerung von einem Magnetelement (14) im proximalen Endbereich der Schubstange und einem antiseriell dazu angeordneten Magnetelement (15) im distalen Endbereich des Linearantriebs (6) gebildet ist.

3. Vorrichtung nach Anspruch 1, **dadurch gekennzeichnet, dass** die Durchdringungsspitze (11) als Bohrer und die Schubstange (13) als Antriebswelle (13) ausgebildet ist, wobei die Antriebswelle (13) mit einem Bohrantrieb (4) in Wirkverbindung steht und der Bohrantrieb (4) in dem Handgerät (2) entlang der Längsachse (L) verschiebbar gelagert und durch den Linearantrieb (6) zur Verschiebung angetrieben ist.

4. Vorrichtung nach Anspruch 3, **dadurch gekennzeichnet, dass** die federnde Lagerung von einem Magnetelement (14) im proximalen Endbereich der Antriebswelle (13) und einem antiseriell dazu angeordneten Magnetelement (15) im distalen Endbereich des Bohrantriebs (4) gebildet ist.

5. Vorrichtung nach Anspruch 2, 3 oder 4, **dadurch gekennzeichnet, dass** die Mittel zum Erkennen des Ausfederns der Schubstange (13) von einem Sensor (19) zum Messen des Magnetfelds der Magnetelemente (14, 15), insbesondere von einem im Bereich des von den Magnetelementen (14, 15) gebildeten Magnetfelds angeordneten Hall-Sensor, gebildet sind.

6. Vorrichtung nach einem der Ansprüche 1 bis 5, **dadurch gekennzeichnet, dass** der Linearantrieb (6) zum automatischen Stoppen des Durchdringungsvorgangs bei Erkennen des Durchdringens der anatomischen Struktur durch Stoppen des Linearantriebs (6) ausgelegt ist.

7. Vorrichtung nach einem der Ansprüche 1 bis 6, **dadurch gekennzeichnet, dass** ein Sensor (19) ein erneutes Einfedern der Schubstange (13) erfasst.

8. Vorrichtung nach einem der Ansprüche 1 bis 7, **dadurch gekennzeichnet, dass** die Schubstange (13) zwischen ihrem proximalen Ende und dem Aufsatz (3) quer zur Längsachse (L) in einen distalen Teil (13b) im Aufsatz (3) und einen proximalen Teil (13a) im Handgerät (2) geteilt ist.

9. Vorrichtung nach einem der Ansprüche 1 bis 8, **dadurch gekennzeichnet, dass** die Durchdringungsspitze (11) hinter einer distalen Spitze (11a) mit einem Hohlraum ausgebildet ist und zumindest eine Durchbrechung (11b) zur Abgabe eines Arzneimittels aus dem Hohlraum aufweist, wobei die Durchdringungsspitze (11) bevorzugt mittels eines Luer/Lock-Anschlusses mit dem distalen Teil (13b) der Schubstange (13) in Wirkverbindung steht.

10. Vorrichtung nach einem der Ansprüche 1 bis 9, **dadurch gekennzeichnet, dass** der Aufsatz (3) aus einem transparenten Kunststoff gebildet ist oder zumindest eine seitliche Durchbrechung aufweist.

11. Vorrichtung nach einem der Ansprüche 3 bis 10, **dadurch gekennzeichnet, dass** der Bohrer (11) zu einer zyklischen Änderung der Drehrichtung antreibbar ist.

12. Vorrichtung nach einem der Ansprüche 1 bis 11, **dadurch gekennzeichnet, dass** der Aufsatz (3) an dem Handgerät (2) durch eine Rastverbindung, eine Drehverbindung oder durch einen Bajonettverschluss festlegbar ist.

13. Vorrichtung nach einem der Ansprüche 1 bis 12, **dadurch gekennzeichnet, dass** das Handgerät (2) hitzefest und druckfest ist.

14. Vorrichtung nach einem der Ansprüche 1 bis 13, **dadurch gekennzeichnet, dass** die Vorrichtung (1) eine Abwurfvorrichtung zum Trennen der Durchdringungsspitze (11) vom Aufsatz (3) aufweist.

## Claims

1. An apparatus (1) for penetrating an anatomical structure, which is designed for detecting the penetration of an anatomical structure and for automatically stopping a penetration process when the penetration of the anatomical structure is detected, wherein, along a longitudinal axis (L) of the apparatus (1), the apparatus (1) comprises a proximal hand-held apparatus (2) and a distal extension attachment (3) detachably fixed thereto, wherein the extension attachment (3) comprises a penetration tip (11) which is mounted displaceably along the longitudinal axis (L) and is operatively connected to a linear drive (6) in the hand-held apparatus (2) via a push rod (13), **characterized in that** the push rod (13) is designed to spring inwards in the proximal direction by means of a resilient bearing, preferably at its proximal end, and the apparatus (1) comprises means for detecting when the push rod (13) springs back outwards in the distal direction when the penetration of the anatomical structure is detected.

2. The apparatus according to claim 1, **characterized in that** the resilient bearing is formed by a magnetic element (14) in the proximal end region of the push rod and a magnetic element (15) arranged antiserially thereto in the distal end region of the linear drive (6).

3. The apparatus according to claim 1, **characterized in that** the penetration tip (11) is designed as a drill and the push rod (13) is designed as a drive shaft (13), wherein the drive shaft (13) is operatively connected to a drill drive (4) and the drill drive (4) is mounted in the hand-held apparatus (2) so as to be displaceable along the longitudinal axis (L) and is driven to displacement by the linear drive (6).

4. The apparatus according to claim 3, **characterized in that** the resilient bearing is formed by a magnetic element (14) in the proximal end region of the drive shaft (13) and a magnetic element (15) arranged antiserially thereto in the distal end region of the drill drive (4).

5. The apparatus according to claim 2, 3 or 4, **characterized in that** the means for detecting when the push rod (13) springs back outwards are formed by a sensor (19) for measuring the magnetic field of the magnetic elements (14, 15), in particular by a Hall sensor arranged in the region of the magnetic field formed by the magnetic elements (14, 15).

6. The apparatus according to any one of claims 1 to 5, **characterized in that** the linear drive (6) is designed to automatically stop the penetration process when the penetration of the anatomical structure is detected by stopping the linear drive (6).

7. The apparatus according to any one of claims 1 to 6, **characterized in that** a sensor (19) detects when the push rod (13) springs inward again.

8. The apparatus according to any one of claims 1 to 7, **characterized in that** the push rod (13) is split, transversely to the longitudinal axis (L), between its proximal end and the extension attachment (3), into a distal part (13b) in the extension attachment (3) and a proximal part (13a) in the hand-held apparatus (2).

9. The apparatus according to any one of claims 1 to 8, **characterized in that** the penetration tip (11) is formed with a cavity behind a distal tip (11a) and comprises at least one through hole (11b) for dispensing a drug from the cavity, the penetration tip (11) preferably being operatively connected to the distal part (13b) of the push rod (13) by means of a Luer-Lock connector.

10. The apparatus according to any one of claims 1 to 9, **characterized in that** the extension attachment (3) is formed from a transparent plastic or at least comprises a lateral through hole.

11. The apparatus according to any one of claims 3 to 10, **characterized in that** the drill (11) can be driven to change the direction of rotation cyclically.

12. The apparatus according to any one of claims 1 to 11, **characterized in that** the extension attachment (3) can be fixed to the hand-held apparatus (2) by a latching connection, a rotary connection, or by a bayonet lock.

13. The apparatus according to any one of claims 1 to 12, **characterized in that** the hand-held apparatus (2) is heat-resistant and pressure-resistant.

14. The apparatus according to any one of claims 1 to 13, **characterized in that** the apparatus (1) comprises an ejection apparatus for separating the penetration tip (11) from the extension attachment (3).

## Revendications

1. Dispositif (1) pour pénétrer une structure anatomique, qui est conçu pour détecter la pénétration d'une structure anatomique et pour arrêter automatiquement un processus de pénétration lors de la détection de la pénétration de la structure anatomique, le dispositif (1) comprenant, le long d'un axe longitudinal (L) du dispositif (1), une pièce à main proximale (2) et un embout distal (3) fixé de manière amovible sur celle-ci, l'embout (3) présentant une pointe de pénétration (11) montée de manière coulissante le long de l'axe longitudinal (L), qui est en liaison fonctionnelle par l'intermédiaire d'une tige de poussée (13) avec un actionneur linéaire (6) dans la pièce à main (2), **caractérisé en ce que** la tige de poussée (13) est conçue, par un montage élastique de préférence à son extrémité proximale, pour se comprimer dans la direction proximale, et le dispositif (1) présente des moyens pour détecter une détente de la tige de poussée dans la direction distale lors de la détection de la pénétration de la structure anatomique.

2. Dispositif selon la revendication 1, **caractérisé en ce que** le montage élastique est formé par un élément magnétique (14) dans la zone d'extrémité proximale de la tige de poussée et par un élément magnétique (15) disposé en anti-série par rapport à celui-ci dans la zone d'extrémité distale de l'actionneur linéaire (6).

3. Dispositif selon la revendication 1, **caractérisé en ce que** la pointe de pénétration (11) est réalisée sous forme de foret et la tige de poussée (13) est réalisée sous forme d'arbre d'entraînement (13), l'arbre d'entraînement (13) étant en liaison fonctionnelle avec un entraînement de perçage (4) et l'entraînement de perçage (4) étant monté de manière coulissante dans la pièce à main (2) le long de l'axe longitudinal (L) et étant entraîné en déplacement par l'actionneur linéaire (6).

4. Dispositif selon la revendication 3, **caractérisé en ce que** le montage élastique est formé par un élément magnétique (14) dans la zone d'extrémité proximale de l'arbre d'entraînement (13) et par un élément magnétique (15) disposé en anti-série par rapport à celui-ci dans la zone d'extrémité distale de l'entraînement de perçage (4).

5. Dispositif selon la revendication 2, 3 ou 4, **caractérisé en ce que** les moyens pour détecter la détente de la tige de poussée (13) sont formés par un capteur (19) pour mesurer le champ magnétique des éléments magnétiques (14, 15), en particulier par un capteur à effet Hall disposé dans la zone du champ magnétique formé par les éléments magnétiques (14, 15).

6. Dispositif selon l'une des revendications 1 à 5, **caractérisé en ce que** l'actionneur linéaire (6) est conçu pour l'arrêt automatique du processus de pénétration lors de la détection de la pénétration de la structure anatomique par l'arrêt de l'actionneur linéaire (6).

7. Dispositif selon l'une des revendications 1 à 6, **caractérisé en ce que** un capteur (19) détecte une nouvelle compression de la tige de poussée (13).

8. Dispositif selon l'une des revendications 1 à 7, **caractérisé en ce que** la tige de poussée (13) est divisée, entre son extrémité proximale et l'embout (3), transversalement à l'axe longitudinal (L) en une partie distale (13b) dans l'embout (3) et une partie proximale (13a) dans la pièce à main (2).

9. Dispositif selon l'une des revendications 1 à 8, **caractérisé en ce que** la pointe de pénétration (11) est réalisée avec une cavité derrière une pointe distale (11a) et présente au moins une ouverture (11b) pour la délivrance d'un médicament depuis la cavité, la pointe de pénétration (11) étant de préférence en liaison fonctionnelle avec la partie distale (13b) de la tige de poussée (13) au moyen d'un raccord Luer-Lock.

10. Dispositif selon l'une des revendications 1 à 9, **caractérisé en ce que** l'embout (3) est formé d'une matière plastique transparente ou présente au moins une ouverture latérale.

11. Dispositif selon l'une des revendications 3 à 10, **caractérisé en ce que** le foret (11) peut être entraîné pour un changement cyclique du sens de rotation.

12. Dispositif selon l'une des revendications 1 à 11, **caractérisé en ce que** l'embout (3) peut être fixé sur la pièce à main (2) par une liaison par encliquetage, une liaison rotative ou par une fermeture à baïonnette.

13. Dispositif selon l'une des revendications 1 à 12, **caractérisé en ce que** la pièce à main (2) est résistante à la chaleur et résistante à la pression.

14. Dispositif selon l'une des revendications 1 à 13, **caractérisé en ce que** le dispositif (1) présente un dispositif d'éjection pour séparer la pointe de pénétration (11) de l'embout (3).
